# EUROPEAN PATENT APPLICATION

(11) **EP 4 822 002 A1**
(43) Date of publication of application: **14.10.2026**
(21) Application number: 24900700.6
(22) Date of filing: 06.12.2024
(51) Int. Cl.: C11B 9/00, A23L 27/00, A61K 8/37, A61Q 13/00, C11D 3/50

(54) **PERFUME COMPOSITION**

(30) Priority: 06.12.2023 US 202363606884 P
(71) Applicant: Takasago International Corporation, Tokyo 144-8721 (JP)
(72) Inventor: NAKAYAMA, Yuji, Hiratsuka-shi, Kanagawa 254-0073 (JP); AIDA, Takashi, Hiratsuka-shi, Kanagawa 254-0073 (JP)
(74) Representative: Hoffmann Eitle
(86) International application number: PCT/JP2024/043192
(87) International publication number: WO 2025/121408

(57) **Abstract**

The present invention relates to a flavor or fragrance composition containing at least one of alkyl-substituted 4-pentenoic acid ester derivatives represented by the general formula (1). In the formula, R₁ to R₃ each independently represent a hydrogen atom or an alkyl group having 1 to 4 carbon atoms, and R₄ represents an alkyl group having 1 to 5 carbon atoms or an alkenyl group having 3 to 5 carbon atoms. The alkyl-substituted 4-pentenoic acid ester derivative is preferably ethyl 4-methyl-4-hexenoate. General Formula (1):

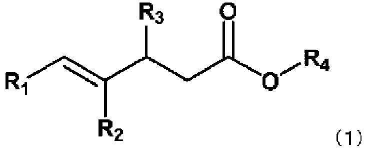

## Description

### TECHNICAL FIELD

The present invention relates to a flavor or fragrance composition, and a fragrance or cosmetic, a toiletry product, a bath agent, a tobacco product, a food or drink, and a pharmaceutical which contain the flavor or fragrance composition.

### BACKGROUND ART

Flavors or Fragrances having a fruity aroma such as a strawberry-like aroma, a green-like aroma, a milky aroma, and a citrus-like aroma have been widely used as flavors or fragrances such as various food flavors, various fragrances or cosmetics, fragrant agents, and other health and hygiene materials. Among these flavors or fragrances having a fruity aroma such as a strawberry-like aroma, a green-like aroma, a milky aroma, and a citrus-like aroma, it is also widely known that carboxylic acid esters of alcohols play an important role as the top note aroma that rises initially (see, for example, Non-Patent Literature 1). However, carboxylic acid esters of alcohols which play an important role as top note aromas of fruity, green-like, and floral fragrances are only those which have already been stereotypical, and for flavorists and a perfumers for designing a fragrance note, a compound exhibiting a new fragrance note different from that of the related art is always desired, in order to characterize the top note and differentiate from an existing flavor or fragrance compound.

### CITATION LIST

### NON-PATENT LITERATURE

Non-Patent Literature 1: Motoichi Indo, Synthetic Fragrances: Chemistry and Product Knowledge (Enlarged and Revised Edition), The Chemical Daily Co., Ltd., March 22, 2005, pp. 461-462

### SUMMARY OF INVENTION

### TECHNICAL PROBLEM

The present invention has been made in view of such circumstances, and an object of the present invention is to provide a compound exhibiting a new fragrance note different from those in the related art, and to provide a flavor or fragrance composition containing the compound and having at least one of a fruity aroma such as a strawberry-like aroma, a green-like aroma, a milky aroma, and a citrus-like aroma.

Another object of the present invention is to provide a fragrance or cosmetic, a toiletry product, a bath agent, a tobacco product, a food or drink, a quasi drug, and a pharmaceutical which contain the flavor or fragrance composition having at least one of a fruity aroma such as a strawberry-like aroma, a green-like aroma, a milky aroma, and a citrus-like aroma.

### SOLUTION TO PROBLEM

Under such circumstances, as a result of intensive studies, the present inventors have found that alkyl-substituted 4-pentenoic acid ester derivatives, in particular, ethyl 4-methyl-4-hexenoate among them has at least any one of an unprecedented excellent fruity aroma such as a strawberry-like aroma, a green-like aroma, a milky aroma, and a citrus-like aroma, has a high natural feeling, and is very useful as a flavor or fragrance material.

That is, the present invention includes the following aspects.
[1] A flavor or fragrance composition containing at least one of alkyl-substituted 4-pentenoic acid ester derivatives represented by the general formula (1): 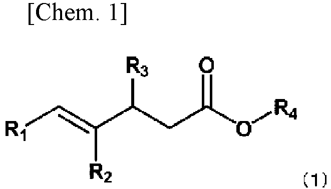 where R₁ to R₃ each independently represent a hydrogen atom or an alkyl group having 1 to 4 carbon atoms, and R₄ represents an alkyl group having 1 to 5 carbon atoms or an alkenyl group having 3 to 5 carbon atoms.
[2] The flavor or fragrance composition according to [1], in which the alkyl-substituted 4-pentenoic acid ester derivative is ethyl 4-methyl-4-hexenoate represented by the following formula (2). 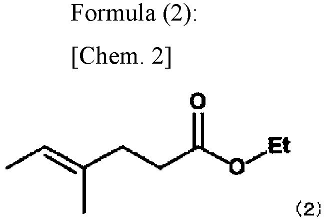
[3] The flavor or fragrance composition according to [1] or [2], in which a blending amount of the alkyl-substituted 4-pentenoic acid ester derivative is 0.001 wt% to 30 wt% based on the entire flavor or fragrance composition.
[4] A fragrance or cosmetic, a toiletry product, a bath agent, a tobacco product, a food or drink, or a pharmaceutical containing 0.0001 wt% to 30 wt% of the flavor or fragrance composition according to any one of [1] to [3].

Incidentally, the synthesis of the alkyl-substituted 4-pentenoic acid ester derivatives can be carried out using a known method in which a corresponding allylic alcohol and a corresponding orthoester are heated in the presence of an acid catalyst to cause the Johnson-Claisen rearrangement. The allyl alcohol as a raw material of the alkyl-substituted 4-pentenoic acid ester derivative can be synthesized according to any known method, such as a reduction reaction of the corresponding enone, a vinyl Grignard reaction to the corresponding aldehyde, and reduction of the corresponding unsaturated carboxylic acid and unsaturated ester.

### ADVANTAGEOUS EFFECTS OF INVENTION

The alkyl-substituted 4-pentenoic acid ester derivatives represented by the general formula (1) of the present invention (in the formula, R₁ to R₃ each independently represent a hydrogen atom or an alkyl group having 1 to 4 carbon atoms, and R₄ represents an alkyl group having 1 to 5 carbon atoms or an alkenyl group having 3 to 5 carbon atoms) and ethyl 4-methyl-4-hexenoate represented by the formula (2) have at least one of a fruity aroma such as a strawberry-like aroma, a green-like aroma, a milky aroma, and a citrus-like aroma, which are clearly different from those similar to those used in the related art, have strong and excellent natural feeling, and are unique. Therefore, the flavor or fragrance composition of the present invention containing these compounds can be effectively used as a flavor for various foods or drinks, various fragrances or cosmetics, toiletry products, bath agents, tobacco products, and pharmaceuticals including health and hygiene materials.

### DESCRIPTION OF EMBODIMENTS

Hereinafter, the present invention will be described in detail.

A flavor or fragrance composition of the present invention contains at least one of alkyl-substituted 4-pentenoic acid ester derivatives represented by the above general formula (1) (in the formula, R₁ to R₃ each independently represent a hydrogen atom or an alkyl group having 1 to 4 carbon atoms, and R₄ represents an alkyl group having 1 to 5 carbon atoms or an alkenyl group having 3 to 5 carbon atoms). The alkyl-substituted 4-pentenoic acid ester derivative is preferably ethyl 4-methyl-4-hexenoate represented by the above formula (2).

The alkyl-substituted 4-pentenoic acid ester derivatives and ethyl 4-methyl-4-hexenoate each have a double bond in a molecule thereof, and therefore, geometric isomers including an (E)-isomer and a (Z)-isomer may be present. The alkyl-substituted 4-pentenoic acid ester derivatives and ethyl 4-methyl-4-hexenoate may be the (E)-isomer alone or the (Z)-isomer alone, or may be a mixture thereof at any ratio.

When the geometric isomers are present, most of the structures of the alkyl-substituted 4-pentenoic acid ester derivatives and ethyl 4-methyl-4-hexenoate are (E)-isomers in terms of a synthesis method. Therefore, these are preferably (E)-alkyl-substituted 4-pentenoic acid ester derivatives and (E)-ethyl 4-methyl-4-hexenoate.

In the formula (1), R₁ to R₃ each represent a hydrogen atom or an alkyl group having 1 to 4 carbon atoms. Examples of the alkyl group having 1 to 4 carbon atoms include a methyl group, an ethyl group, a propyl group, an isopropyl group, a butyl group, a 2-methylpropyl group, a 1-methylpropyl group, and a 1,1-dimethylethyl group, and R₁ to R₃ in the present invention are limited thereto. R₁ is preferably a hydrogen atom, a methyl group, an ethyl group, a propyl group, or a butyl group, and more preferably a methyl group. R₂ is preferably a hydrogen atom or a methyl group, and more preferably a methyl group. R₃ is preferably a hydrogen atom or a propyl group, and more preferably a hydrogen atom.

In the formula (1), R₄ is an acid residue of an ester, and is an alkyl group having 1 to 5 carbon atoms or an alkenyl group having 3 to 5 carbon atoms. Examples of the alkyl group having 1 to 5 carbon atoms include a methyl group, an ethyl group, a propyl group, an isopropyl group, a butyl group, a 2-methylpropyl group, a 1-methylpropyl group, a 1,1-dimethylethyl group, a pentyl group, a 1-methylbutyl group, a 2-methylbutyl group, a 3-methylbutyl group, a 1,1-dimethylpropyl group, a 1,2-dimethylpropyl group, a 2,2-dimethylpropyl group, and a 1-ethylpropyl group. Examples of the alkenyl group having 3 to 5 carbon atoms include an allyl group, a 1-methylallyl group, a 2-methylallyl group, a 2-butenyl group, a 3-butenyl group, a 1,1-dimethylallyl group, a 1,2-dimethylallyl group, a 1-methyl-2-butenyl group, a 2-methyl-2-butenyl group, a 3-methyl-2-butenyl group, a 3-pentenyl group, a 1-methyl-3-butenyl group, a 2-methyl-3-butenyl group, a 3-methyl-3-butenyl group, and a 4-pentenyl group. R₄ in the present invention is limited to these. R₄ is preferably an ethyl group, an isopropyl group, or a butyl group, and more preferably an ethyl group.

Examples of the alkyl-substituted 4-pentenoate derivative represented by the general formula (1) include ethyl 4-methyl-4-hexenoate, ethyl 4-octenoate, ethyl 4-methyl-4-heptenoate, ethyl 4-methyl-4-nonenoate, ethyl 3-vinylhexanoate, isopropyl 4-methyl-4-hexenoate, and butyl 4-methyl-4-hexenoate. Among these, ethyl 4-methyl-4-hexenoate is particularly preferred.

Those in which these geometric isomers are present can also be used in the form of one of the isomers or a mixture of both isomers at any ratio. Further, in the case where an optical isomer is present, one of the isomers or a mixture of both isomers at any ratio can be used.

The alkyl-substituted 4-pentenoic acid ester derivatives represented by the above general formula (1) are obtained by heating a mixture of alkyl-substituted allylic alcohols represented by the following general formula (3): 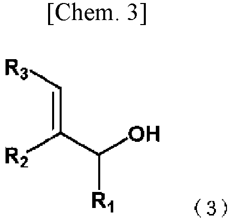 (in the formula, R₁ to R₃ each independently represent a hydrogen atom or an alkyl group having 1 to 4 carbon atoms) and trialkyl orthoacetate (where the alkyl group is generally a saturated hydrocarbon group having 1 to 4 carbon atoms), more preferably triethyl orthoacetate in the presence of an acid catalyst to cause known Johnson-Claisen rearrangement and performing hydrolysis and esterification if necessary.

Examples of the acid catalyst necessary for causing the Johnson-Claisen rearrangement include, but are not particularly limited to, carboxylic acids such as acetic acid, propionic acid, butyric acid, isobutyric acid, and valeric acid, sulfonic acids such as benzenesulfonic acid, toluenesulfonic acid, methanesulfonic acid, and trifluoromethanesulfonic acid, and inorganic acids such as phosphoric acid and sulfuric acid.

The Johnson-Claisen rearrangement is a reaction generally proceeding at a reaction temperature of 50°C to 280°C, more preferably 90°C to 180°C under normal pressure. However, when the reaction is performed under a high pressure or in a gas phase reaction or the like, the temperature range is not limited thereto.

As a by-product of the Johnson-Claisen rearrangement reaction, in addition to the above alkyl-substituted 4-pentenoic acid ester derivative which is a target product, an ester having the alkyl-substituted allylic alcohol used as a raw material of the reaction as an ester acid residue may be simultaneously produced.

For example, when an allyl alcohol represented by the following formula (4) 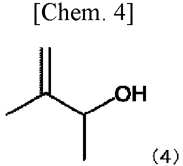 is used as a raw material and triethyl orthoacetate is caused to react in the presence of an acid catalyst, ethyl 4-methyl-4-hexenoate represented by the following formula (2) 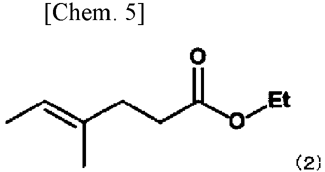 is generated, and further, 4-methyl-4-hexenoic acid-3-methyl-3-buten-2-yl represented by the following formula (5) 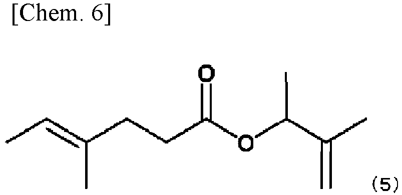 is generated. However, even though 4-methyl-4-hexenoic acid-3-methyl-3-buten-2-yl is contained, the aroma is not particularly affected.

In addition, when the raw material represented by the general formula (3) is used, a similar by-product is generated, but when a molecular weight of the by-product exceeds a molecular weight of the compound represented by the above formula (5), no aroma is felt.

The alkyl-substituted 4-pentenoic acid ester derivatives represented by the general formula (1) and ethyl 4-methyl-4-hexenoate represented by the formula (2) of the present invention have at least one of a fruity aroma such as a strawberry-like aroma, a green-like aroma, a milky aroma, and a citrus-like aroma, which are clearly different from the commonly used compound having a fruity aroma or a green-like aroma, have strong and excellent natural feeling, and are unique. Therefore, they may be used alone or in combination of two or more kinds thereof as aroma components, but in general, the alkyl-substituted 4-pentenoic acid ester derivatives or ethyl 4-methyl-4-hexenoate is blended with commonly known or well-known ketones, aldehydes, esters, alcohols, ethers, terpenes, natural essential oils, synthetic musks, and the like as aroma components alone or in combination as additional flavor or fragrance components to obtain a flavor or fragrance composition. The additional flavor or fragrance component is blended in the flavor or fragrance composition in an amount of 0.001 wt% to 99.999 wt%, 0.001 wt% to 99.9 wt%, 0.01 wt% to 99 wt%, 0.1 wt% to 99 wt%, 0.1 wt% to 90 wt%, 0.1 wt% to 80 wt%, 1 wt% to 70 wt%, 2 wt% to 60 wt%, 5 wt% to 50 wt%, 10 wt% to 40 wt%, 15 wt%% to 30 wt%, or 20 wt% to 25 wt%.

The flavor or fragrance composition of the present invention may further contain a solvent.

Non-limiting examples of the solvent include dipropylene glycol, propylene glycol, diethyl phthalate (DEP), diisononyl phthalate (DINP), benzyl benzoate, benzyl alcohol, isopropyl myristate (IPM), isopropyl palmitate (IPP/Deltyl Prim), butyl stearate, dioctyl adipate, triethyl citrate, methyl hydrogenated rosinate (CAS No. 8050-15-5), terpene (for example, Glidsol 100), a paraffin-based naphthenic solvent (for example, a LPA-170 solvent), an isoalkane (for example, Soltrol 170 isoparaffin), isoparaffin, isooctadecanol (for example, Tego Alkanol 66), phenoxyethanol, diethylene glycol monoethyl ether (Carbitol Low Gravity), glycol ether (methyl carbitol), dipropylene glycol methyl ether (for example, Dowanol DPM), dipropylene glycol methyl ether acetate (for example, Dowanol DPMA), propylene glycol methyl ether (for example, Dowanol PM glycol ether), tripropylene glycol methyl ether, diisoheptyl phthalate (for example, Jayflex (registered trademark) 77 available from Exxon), deionized water or distilled water, in particular, modified ethyl alcohol (for example, SDA40B), dimethyl adipate/dimethyl glutamate (for example, DBE (registered trademark)-LVP ester available from FLEXISOLV (registered trademark), a racemic mixture (+/-)-2,2-dimethyl-4-hydroxymethyl-1,3-dioxolane (for example, Augo Clean Multi Solvent), Alcohol 40B Anhydrous 200 Proof, Alcohol SDA 40B 190 Proof, triacetin, 3-methoxy-3-methyl-1-butanol (Solfit), benzyl laurate, tripropylene glycol methyl ether (for example, Dowanol TPM), dipropylene glycol n-butyl ether (for example, Dowanol DPNB), dimethylsiloxane, trimethylsiloxy-terminated solution (for example, Dowanol Corning 200 solution), caprylic/capric triglyceride (for example, Neobee M-5), propylene glycol and glyceryl oleate (for example, Arlacel 186), Uniceth-IC20L (for example, Arlasolve 200L), 1,3-propanediol, butyl cellosolve, hexylene glycol, glycerin, N-methyl stearate, isopropyl alcohol, 2-methyl-1,3-propanediol (for example, MP Diol Glycol), diethyl citrate, triethyl acetyl citrate, isopentyl diacetate (IPD-AC), dimethyl 2-methylpentanediate (for example, Rhodiasolve Iris), medium-chain triglycerides (MTC), terpene-based hydrocarbons (for example, Dipentene 5100), DL-limonene (for example, Dipentene 122), 3,5,5-trimethylhexyl acetate, diethyl malonate, limonene (for example, Unitene D), cyclohexyl acetate, para-tert-butyl ether (for example, Vertenex), ethyl acetate, diethyl succinate, and natural solvents (including, but not limited to, vegetable oils such as sunflower oil).

The blending amount of the alkyl-substituted 4-pentenoic acid ester derivative and ethyl 4-methyl-4-hexenoate in the flavor or fragrance composition of the present invention is set to an appropriate amount depending on the flavor or fragrance composition to which the above compounds are applied or the form, usage, and the like of a product to which the flavor or fragrance composition is further applied. In general, the alkyl-substituted 4-pentenoic acid ester derivative and 4-methyl-4-hexenoic acid ethyl are preferably blended in the flavor or fragrance composition in an amount of 0.001 wt% to 30 wt%, and particularly preferably blended in the flavor or fragrance composition in an amount of 0.01 wt% to 10 wt%.

The flavor or fragrance composition containing the alkyl-substituted 4-pentenoic acid ester derivatives represented by the general formula (1) or the ethyl 4-methyl-4-hexenoate represented by the formula (2) can be suitably used as a flavor or fragrance used in various products such as fragrances or cosmetics, toiletry products, bath agents, tobacco products, foods or drinks, and pharmaceuticals. The blending amount and application method of the flavor or fragrance composition obtained by the present invention to a product can be appropriately changed depending on the type of the product, the purpose of use, and the like. Generally, the blending amount of the flavor or fragrance composition into the product is 0.0001 wt% to 30 wt%, preferably 0.001 wt% to 10 wt% in a final product composition.

The fragrances or cosmetics, toiletry products, bath agents, tobacco products, foods or drinks, and pharmaceuticals to which the flavor or fragrance composition of the present invention is applied will be described in more detail.

Examples of the fragrances or cosmetics to which the flavor or fragrance composition of the present invention is applied include a fragrance product, a foundation cosmetic, a finishing cosmetic, a hair cosmetic, a suntan cosmetic, a medicated cosmetic, a hair care product, a soap, a body washer, a detergent, a soft finishing agent, a cleaner, a kitchen cleaner, a bleaching agent, an aerosol agent, a deodorant or fragrant agent, a repellent, and other miscellaneous goods.

More specifically,
as the fragrance product, examples thereof include a perfume, eau de parfum, eau de toilette, eau de cologne, and the like;
as the foundation cosmetic, examples thereof include a facial cleansing cream, a vanishing cream, a cleansing cream, a cold cream, a massage cream, a milky lotion, a skin lotion, a beauty lotion, a facial pack, a makeup remover, and the like;
as the finishing cosmetic, examples thereof include a foundation, a face powder, a solid face powder, a talcum powder, a rouge, a lip balm, a cheek rouge, an eye liner, a mascara, an eye shadow, an eyebrow pencil, an eye pack, a nail enamel, an enamel remover, and the like; and
as the hair cosmetic, examples thereof include a pomade, a brilliantine, a hair set lotion, a hair stick, a hair solid, a hair oil, a hair treatment, a hair cream, a hair tonic, a hair liquid, a hair spray, a bandoline, a revitalizing hair tonic, a hair dye, and the like.

As the suntan cosmetic, examples thereof include a suntan product, a sun-screen product, and the like;
as the medicated cosmetic, examples thereof include an antiperspirant, an after-shaving lotion or gel, a permanent wave agent, a medicated soap, a medicated shampoo, a medicated skin cosmetic, and the like;
as the hair care product, examples thereof include a shampoo, a rinse, a rinse-in-shampoo, a conditioner, a treatment, a hair pack, and the like;
as the soap, examples thereof include a toilet soap, a bath soap, a perfume soap, a transparent soap, a synthetic soap, and the like;
as the body washer, examples thereof include a body soap, a body shampoo, a hand soap, and the like; and
as the detergent, examples thereof include a heavy detergent for clothing, a light detergent for clothing, a liquid detergent, a washing soap, a compact detergent, a powder soap, and the like.

As the soft finishing agent, examples thereof include a softener, a furniture care, and the like;
as the cleaning agent, examples thereof include a cleanser, a house cleaner, a toilet cleaner, a bath cleaner, a glass cleaner, a mildew remover, a cleaner for drainpipe use, and the like;
as the kitchen cleaner, examples thereof include a kitchen soap, a kitchen synthetic soap, a tableware cleaner, and the like;
as the bleaching agent, examples thereof include an oxidation type bleaching agent (such as a chlorine-based bleaching agent and an oxygen-based bleaching agent), a reduction type bleaching agent (such as a sulfur-based bleaching agent), an optical bleaching agent, and the like;
as the aerosol agent, examples thereof include a spray type one, a powder spray, and the like;
as the deodorant or fragrant agent, examples thereof include a solid type one, a gel type one, a liquid type one, and the like;
as the repellent, examples thereof include a mist spray type one, an aqueous liquid type one, and the like; and
as the miscellaneous goods, examples thereof include tissue paper, toilet paper, and the like.

In addition, as the toiletry product to which the flavor or fragrance composition of the present invention is applied, examples thereof include a toothpaste, an oral cavity cleaner, a mouthwash, a shaving cream, and a skin lotion.

As the bath agent to which the flavor or fragrance composition of the present invention is applied, examples thereof include bath agents (a bath salt, a bath tablet, a bath liquid, etc.), a foam bath (a bubble bath, etc.), bath oil (a bath perfume, a bath capsule, etc.), a milk bath, a bath jelly, a bath cube, and the like.

As the tobacco product to which the flavor or fragrance composition of the present invention is applied, examples thereof include a cigarette, a cigar, a pipe, Kiseru, Hookah, smokeless tobacco products, a heated tobacco product, an electronic cigarette, and the like.

As the food or drink to which the flavor or fragrance composition of the present invention is applied, examples thereof include drinks such as fruit juice drinks, fruit wines, milk drinks, carbonated drinks, soft drinks, and healthy drinks; ices such as ice creams, sherbets, and ice candies; desserts such as jelly and pudding; western style confections such as cakes, cookies, chocolates, and chewing gum; Japanese style confections such as bean-jam bun, sweet bean jelly, and uiro; jams; candies; breads; tea drinks or luxury drinks such as green tea, Oolong tea, black tea, persimmon leaf tea, chamomile tea, low striped bamboo tea, mulberry tea, dokudami tea, Pu-er tea, mate tea, Rooibos tea, Gymnema tea, Guava tea, coffee, and cocoa; soups such as Japanese style soup, Western style soup, and Chinese soup; flavored seasonings; various instant drinks and foods; various snack foods; and the like.

As the pharmaceutical to which the flavor or fragrance composition of the present invention is applied, examples thereof include external skin preparations such as poultices and ointments, oral preparations, medicated cosmetics, medicated lotions, and the like.

However, the products to which the flavor or fragrance composition of the present invention can be applied are not limited to those described above.

Furthermore, components according to the purpose of use of each product are appropriately blended in the product to which the component is applied, and the product is provided as a fragrance or cosmetic, a toiletry product, a bath agent, a tobacco product, a food or drink, a pharmaceutical, or the like. In addition, the alkyl-substituted 4-pentenoic acid ester derivative represented by the general formula (1) or the ethyl 4-methyl-4-hexenoate represented by the formula (2) is preferably blended in a state of being contained in the flavor or fragrance composition when blended in a product. Alternatively, the alkyl-substituted 4-pentenoic acid ester derivative or the ethyl 4-methyl-4-hexenoate and the flavor or fragrance composition not containing them can also be blended as separate blending components.

### Examples

Hereinafter, the present invention will be specifically described with reference to Synthesis Examples and Examples, but the present invention is not limited thereto.

In the Synthesis Examples below, the nuclear magnetic resonance spectrum (NMR) and the mass spectrum (MS) were measured by the following apparatus.
Nuclear magnetic resonance spectrum (¹H NMR, ¹³C NMR): DRX-500 type apparatus (manufactured by Bruker Corporation)
Mass spectrum (MS): GCMS-QP2010 type apparatus (manufactured by Shimadzu Corporation)

### [Synthesis Example 1]

### (Synthesis of ethyl 4-methyl-4-hexenoate)

In a 500 ml (capacity) reaction flask equipped with a thermometer, a Dean-Stark apparatus, and a reflux condenser were added 103.4 g of 3-methyl-3-buten-2-ol (molecular weight: 86.13, 1.20 mol), 253.1 g of triethyl orthoacetate (molecular weight: 162.23, 1.56 mol), and 0.5 g of propionic acid (molecular weight: 74.08, 6.75 mmol), followed by heating at an oil bath temperature of 180°C in a nitrogen stream, and heating was performed for 4 hours while removing generated ethanol from the Dean-Stark apparatus.

Unreacted 3-methyl-3-buten-2-ol and triethyl orthoacetate were distilled off by a rotary evaporator and then, 154.11 g (molecular weight: 156.23, 0.98 mol) of target ethyl 4-methyl-4-hexenoate was obtained as a colorless, transparent, oily product by distillation under reduced pressure. (Yield: 82.2%)
bp.70°C to 72°C/1400 Pa

The measurement values of MS, ¹H NMR, and ¹³C NMR of the obtained compound were as follows.
GC/MS (m/e): 156 (M+), 125, 110, 83, 82, 69, 67, 55, 43, 41, 40, 29, 27.
¹H NMR (500 MHz, CDCl₃): δ = 5.23 (m, 1H), 4.12 (q, J = 7.2 Hz, 2H), 2.40-2.32 (m, 2H), 2.32-2.25 (m, 2H), 1.61 (s, 3H), 1.58-1.54 (m, 3H), 1.25 (t, J = 7.2 Hz, 3H).
¹³C NMR (125 MHz, CDCl₃): δ = 173.56 (s), 134.01 (d), 119.19 (d), 60.21 (t), 34.65 (q), 33.19 (q), 15.52 (t), 14.22 (t), 13.32 (q).

### [Synthesis Example 2]

### (Synthesis of Ethyl 4-octenoate]

In a 500 ml (capacity) reaction flask equipped with a thermometer, a Dean-Stark apparatus, and a reflux condenser were added 100.2 g of 1-hexen-3-ol (molecular weight: 100.16, 1.00 mol), 210.9 g of triethyl orthoacetate (molecular weight: 162.23, 1.30 mol), and 0.4 g of propionic acid (molecular weight: 74.08, 5.40 mmol), followed by heating at an oil bath temperature of 180°C in a nitrogen stream, and heating was performed for 4 hours while removing the generated ethanol from the Dean-Stark apparatus.

Unreacted 1-hexen-3-ol and triethyl orthoacetate were distilled off by a rotary evaporator, and then, 133.82 g of target ethyl 4-octenoate (molecular weight: 170.25, 0.79 mol) was obtained as a colorless, transparent, oily product by distillation under reduced pressure. (Yield: 78.6%)
bp.85°C to 87°C/1400 Pa

The measurement values of MS, ¹H NMR, and ¹³C NMR of the obtained compound were as follows.
GC/MS (m/e): 170 (M+), 125, 124, 96, 88, 84, 82, 81, 71, 70, 67, 55, 43, 41, 29, 27.
¹H NMR (500 MHz, CDCl₃): δ = 5.50-5.35 (m, 4H), 4.12 (q, J = 7.2 Hz, 2H), 2.38-2.27 (m, 2H), 1.94 (q, J = 7.2 Hz, 2H), 1.40-1.31 (m, 4H), 1.24 (t, J = 7.2 Hz, 3H), 0.87 (t, J = 7.4 Hz, 3H).
¹³C NMR (125 MHz, CDCl₃): δ = 173.38 (s), 131.57 (d), 128.12 (d), 60.21 (t), 34.57 (t), 34.45 (t), 27.95 (t), 22.54 (t), 14.24 (q), 13.58 (q).

### [Synthesis Example 3]

### (Synthesis of Ethyl 4-methyl-4-heptenoate)

In a 500 ml (capacity) reaction flask equipped with a thermometer, a Dean-Stark apparatus, and a reflux condenser were added 100.2 g of 2-methyl-1-penten-3-ol (molecular weight: 100.16, 1.00 mol), 210.9 g of triethyl orthoacetate (molecular weight: 162.23, 1.30 mol), and 0.4 g of propionic acid (molecular weight: 74.08, 5.40 mmol), followed by heating at an oil bath temperature of 180°C in a nitrogen stream, and heating was performed for 4 hours while removing generated ethanol from the Dean-Stark apparatus.
Unreacted 2-methyl-1-penten-3-ol and triethyl orthoacetate were distilled off by a rotary evaporator, and then, 130.07 g of target ethyl 4-methyl-4-heptenoate (molecular weight: 170.25, 0.76 mol) was obtained as a colorless, transparent, oily product by distillation under reduced pressure. (Yield: 76.4%)
bp.82°C to 83°C/1300 to 1400 Pa

The measurement values of MS, ¹H NMR, and ¹³C NMR of the obtained compound were as follows.
GC/MS (m/e): 170 (M+), 125, 124, 97, 96, 95, 88, 83, 82, 81, 70, 69, 67, 55, 41, 29, 27.
¹H NMR (500 MHz, CDCl₃): δ = 5.15 (dt, J = 1.3 Hz, J = 7.1 Hz, 1H), 4.11 (q, J = 7.2, 2H), 2.40 (dt, J = 1.3 Hz, J = 7.7 Hz, 2H), 2.29 (t, J = 7.7 Hz, 2H), 1.98 (dq, J = 7.1, J = 7.6, 2H), 1.60 (s, 3H), 1.24 (t, J = 7.2, 3H), 0.92 (t, J = 7.6, 3H).
¹³C NMR (125 MHz, CDCl₃): δ = 173.53 (s), 132.60 (s), 127.20 (d), 60.19 (t), 34.65 (t), 33.26 (t), 21.12 (t), 15.70 (q), 14.24 (q).

### [Synthesis Example 4]

### (Synthesis of Ethyl 4-methyl-4-nonenoate)

In a 500 ml (capacity) reaction flask equipped with a thermometer, a Dean-Stark apparatus, and a reflux condenser were added 128.2 g of 2-methyl-1-penten-3-ol (molecular weight: 128.22, 1.00 mol), 210.9 g of triethyl orthoacetate (molecular weight: 162.23, 1.30 mol), and 0.4 g of propionic acid (molecular weight: 74.08, 5.40 mmol), followed by heating at an oil bath temperature of 180°C in a nitrogen stream, and heating was performed for 4 hours while removing generated ethanol from the Dean-Stark apparatus.
Unreacted 2-methyl-1-penten-3-ol and triethyl orthoacetate were distilled off by distillation, and then, 148.34 g of target ethyl 4-methyl-4-nonenate (molecular weight: 198.31, 0.75 mol) was obtained as a colorless, transparent, oily product by distillation under reduced pressure. (Yield: 74.8%)
bp.94°C to 96°C/700 Pa

The measurement values of MS, ¹H NMR, and ¹³C NMR of the obtained compound were as follows.
GC/MS (m/e): 198 (M+), 153, 142, 124, 111, 110, 109, 96, 95, 88, 81, 70, 69, 68, 67, 55, 43, 41, 29, 27.
¹H NMR (500 MHz, CDCl₃): δ = 5.16 (dt, J = 1.2 Hz, J = 7.2 Hz, 1H), 4.11 (q, J = 7.2, 2H), 2.40 (dt, J = 1.20 Hz, J = 8.2 Hz, 2H), 2.29 (t, J = 8.2 Hz, 2H), 2.01-1.93 (m, 2H), 1.60 (s, 3H), 1.33-1.26 (m, 4H), 1.24 (t, J = 7.2, 3H), 0.94-0.85 (m, 3H).
¹³C NMR (125 MHz, CDCl₃): δ = 173.54 (s), 133.07 (s), 125.59 (d), 60.20 (t), 34.72 (t), 33.30 (t), 31.90 (t), 27.55 (t), 22.28 (t), 15.85 (q), 14.24 (q), 13.99 (q).

### [Synthesis Example 5]

### (Synthesis of Ethyl 3-vinylhexanoate)

In a 500 ml (capacity) reaction flask equipped with a thermometer, a Dean-Stark apparatus, and a reflux condenser were added 100.2 g of 2-hexen-3-ol (molecular weight: 100.16, 1.00 mol), 210.9 g of triethyl orthoacetate (molecular weight: 162.23, 1.30 mol), and 0.4 g of propionic acid (molecular weight: 74.08, 5.40 mmol), followed by heating at an oil bath temperature of 180°C in a nitrogen stream, and heating was performed for 4 hours while removing the generated ethanol from the Dean-Stark apparatus.
Unreacted 2-hexen-3-ol and triethyl orthoacetate were distilled off by distillation, and then, 139.26 g of target ethyl 3-vinylhexanoate (molecular weight: 170.25, 0.82 mol) was obtained as a colorless, transparent, oily product by distillation under reduced pressure. (Yield: 81.8%)
bp.76°C to 78°C/1600 Pa

The measurement values of MS, ¹H NMR, and ¹³C NMR of the obtained compound were as follows.
GC/MS (m/e): 170 (M+), 155, 141, 127, 124, 96, 88, 83, 81, 68, 43, 41, 29, 27.
¹H NMR (500 MHz, CDCl₃): δ = 5.56 (ddd, J = 10.2 Hz, J = 8.4 Hz, J = 17.2 Hz, 1H), 5.05-4.97 (m, 2H), 4.12 (q, J = 7.1 Hz, 2H), 2.35 (dd, J = 6.2 Hz, J = 7.3 Hz, 1H), 2.26 (dd, J = 8.4 Hz, J = 7.3 Hz, 1H), 1.40-1.20 (m, 4H), 1.24 (t, J = 7.1 Hz, 3H), 0.89 (t, J = 6.8 Hz, 3H).
¹³C NMR (125 MHz, CDCl₃): δ = 172.61 (s), 141.13 (d), 114.86 (t), 60.15 (t), 40.24 (d), 40.16 (t), 36.68 (t), 20.04 (t), 14.27 (q), 13.96 (q).

### [Synthesis Example 6]

### (Synthesis of 4-methyl-4-hexenoic acid)

In a 200 ml (capacity) reaction flask equipped with a thermometer were added 31.25 g of ethyl 4-methyl-4-hexenoate (molecular weight: 156.23, 0.20 mol) obtained in Synthesis Example 1 and a 30% sodium hydroxide aqueous solution (sodium hydroxide: 16.00 g, (molecular weight: 40.0, 0.4 mol)), followed by stirring at room temperature. The reaction was monitored by a gas chromatograph, and ethyl 4-methyl-4-hexenoate as a raw material disappeared in 2 hours after the stirring. The organic layer was removed, the remaining material was washed twice with hexane and then neutralized by adding 20% sulfuric acid (sulfuric acid: 19.60 g, (molecular weight: 98.0, 0.2 mol)), the organic layer was extracted twice with ethyl acetate and washed twice with water, and then, the solvent was distilled off with a rotary evaporator to obtain 24.84 g of 4-methyl-4-hexenoic acid (molecular weight: 128.17) as a pale yellow, transparent, oily product. This crude was directly used in the following esterification reaction.

### [Synthesis Example 7]

### (Synthesis of Isopropyl 4-methyl-4-hexenoate)

In a 50 ml (capacity) reaction flask equipped with a thermometer were added 20 ml of toluene, 1.0 g of 4-methyl-4-hexenoic acid (molecular weight: 128.17, 7.8 mmol) obtained in Synthesis Example 6, 938 mg of isopropanol (molecular weight: 60.10, 15.6 mmol), and 3.22 g of dicyclohexylcarbodiimide (molecular weight: 206.33, 15.6 mmol), followed by stirring at room temperature for 3 hours. The resulting salt was filtered, the solvent was then distilled off with a rotary evaporator under reduced pressure, and then, the residue was purified by silica gel chromatography to obtain 1.12 g of target isopropyl 4-methyl-4-hexenoate (molecular weight: 170.25, 6.58 mol) as a colorless, transparent, oily product. (Yield: 84.3%)

The measurement values of MS, ¹H NMR, and ¹³C NMR of the obtained compound were as follows.
GC/MS (m/e): 170 (M+), 128, 113, 111, 110, 85, 83, 82, 69, 67, 55, 43, 41, 29, 27.
¹H NMR (500 MHz, CDCl₃): δ = 5.23 (dq, J = 6.6 Hz, J = 1.1 Hz, 1H), 5.00 (sep, J = 6.3 Hz, 1H), 2.40-2.32 (m, 2H), 2.32-2.25 (m,2H), 1.61 (s, 3H), 1.56 (d, J = 6.7 Hz, 3H), 1.22 (d, J = 6.3 Hz, 6H).
¹³C NMR (125 MHz, CDCl₃): δ = 173.07 (s), 134.06 (s), 119.20 (d), 67.38 (d), 34.77 (t), 33.51 (t), 21.83 (q), 15.50 (q), 13.30 (q).

### [Synthesis Example 8]

### (Synthesis of Butyl 4-methyl-4-hexenoate)

In a 50 ml (capacity) reaction flask equipped with a thermometer were added 20 ml of toluene, 1.0 g of 4-methyl-4-hexenoic acid (molecular weight: 128.17, 7.8 mmol) obtained in Synthesis Example 6, 1.16 g of 1-butanol (molecular weight: 74.12, 15.6 mmol), and 3.22 g of dicyclohexylcarbodiimide (molecular weight: 206.33, 15.6 mmol), followed by stirring at room temperature for 3 hours. The resulting salt was filtered, the solvent was then distilled off with a rotary evaporator under reduced pressure, and then, the residue was purified by silica gel chromatography to obtain 1.19 g of target butyl 4-methyl-4-hexenoate (molecular weight: 184.28, 6.46 mol) as a colorless, transparent, oily product. (Yield: 82.8%)

The measurement values of MS, ¹H NMR, and ¹³C NMR of the obtained compound were as follows.
GC/MS (m/e): 184 (M+), 128, 111, 110, 95, 85, 83, 82, 69, 67, 57, 55, 43, 41, 39, 29, 27.
¹H NMR (500 MHz, CDCl₃): δ = 5.23 (dq, J = 6.6 Hz, J = 1.1 Hz, 1H), 4.06 (t, J = 6.7 Hz, 2H), 2.43-2.35 (m, 2H), 2.35-2.26 (m, 2H), 1.66-1.52 (m, 8H), 1.38 (td, J = 7.4 Hz, J = 14.9 Hz, 2H), 0.93 (t, J = 7.4 Hz, 3H).
¹³C NMR (125 MHz, CDCl₃): δ = 173.64 (s), 134.04 (s), 119.19 (d), 64.14 (t), 34.70 (t), 33.21 (t), 30.70 (t), 19.21 (t), 15.52 (q), 13.69 (q), 13.32 (q).

### [Example 1]

### (Sensory Evaluation)

The odor of each of ethyl 4-methyl-4-hexenoate, ethyl 4-octenoate, ethyl 4-methyl-4-heptenoate, ethyl 4-methyl-4-nonenoate, ethyl 3-vinylhexanoate, isopropyl 4-methyl-4-hexenoate, and butyl 4-methyl-4-hexenoate obtained in Synthesis Examples 1 to 5 and 7 and 8 was evaluated on a blotter by seven expert panelists. The results are shown in Table 1.

**[Table 1]**

| Compound name | Evaluation results |
|---|---|
| Ethyl 4-methyl-4-hexenoate | Milky, strawberry-like, and fruity odor |
| Ethyl 4-octenoate | Metallic note, citrus-like, and fruity odor |
| Ethyl 4-methyl-4-heptenoate | Metallic note, citrus-like, and fruity odor |
| Ethyl 4-methyl-4-nonenoate | Metallic note, citrus-like, and fruity odor |
| Ethyl 3-vinylhexanoate | Citrus-like, strawberry-like, and fruity odor |
| Isopropyl 4-methyl-4-hexenoate | Milky, strawberry-like, and fruity odor |
| Butyl 4-methyl-4-hexenoate | Milky, strawberry-like, and fruity odor |

### [Example 2 and Comparative Example 1]

Using ethyl 4-methyl-4-hexenoate obtained in Synthesis Example 1, 100 g of a fruity strawberry-like fragrance composition was prepared according to the following formulation (Example 2). In addition, 100 g of a flavor or fragrance composition without ethyl 4-methyl-4-hexenoate was prepared (Comparative Example 1). The odor of these flavor or fragrance compositions were evaluated by seven expert panelists. In the table, the unit of the blending amount is g.

**[Table 2]**

| Components | Example 2 | Comparative Example 1 |
|---|---|---|
| Ambretone | 50.0 | 50.0 |
| γ-undecalactone | 25.0 | 25.0 |
| Ethyl 4-methyl-4-hexenoate | 1.0 | - |
| 1%Labdanum oil in DPG solution | 2.5 | 2.5 |
| Dipropylene glycol (DPG) | 21.5 | 22.5 |
| Total | 100.0 | 100.0 |

As a result of the evaluation, all the seven expert panelists reported that the flavor or fragrance composition in Example 2 exhibited a strawberry fruity green-like note with higher palatability and a more natural feeling than the composition in Comparative Example 1.

### [Example 3 and Comparative Example 2]

### (Strawberry Flavor)

Using ethyl 4-methyl-4-hexenoate obtained in Synthesis Example 1, a flavor or fragrance composition (strawberry flavor) was prepared according to the following formulation (Example 3). In addition, a flavor or fragrance composition (strawberry flavor) without ethyl 4-methyl-4-hexenoate was prepared (Comparative Example 2). The odor of these flavor or fragrance compositions were evaluated by seven expert panelists. In the table, the unit of the blending amount is part(s) by weight.

**[Table 3]**

| Components | Example 3 | Comparative Example 2 |
|---|---|---|
| Ethyl 4-methyl-4-hexenoate | 20.000 | - |
| Acetoin | 0.250 | 0.250 |
| Isoamyl acetate | 70.000 | 70.000 |
| Isoamyl butyrate | 30.000 | 30.000 |
| Isoamyl isovalerate | 15.000 | 15.000 |
| n-butyl butyrate | 15.000 | 15.000 |
| Isobutyl butyrate | 15.000 | 15.000 |
| Ethyl acetate | 10.000 | 10.000 |
| Ethyl butyrate | 20.000 | 20.000 |
| Hexanal | 0.200 | 0.200 |
| trans-2-hexanal | 0.400 | 0.400 |
| cis-3-hexenal | 3.000 | 3.000 |
| δ-dodecalactone | 0.100 | 0.100 |
| Butyric acid | 0.050 | 0.050 |
| Isovaleric acid | 0.050 | 0.050 |
| Propylene glycol (solvent) | 800.950 | 820.950 |
| Total | 1000.000 | 1000.00 |

As a result of the evaluation, all the seven expert panelists reported that the flavor or fragrance composition in Example 3 exhibited a strawberry-like fragrance note with a higher palatability and a higher natural feeling than the flavor or fragrance composition in Comparative Example 2.

### [Example 4 and Comparative Example 3]

### (Mango Flavor)

Using ethyl 4-methyl-4-hexenoate obtained in Synthesis Example 1, a flavor or fragrance composition (mango flavor) was prepared according to the following formulation (Example 4). In addition, a flavor or fragrance composition (mango flavor) containing no ethyl 4-methyl-4-hexenoate was prepared (Comparative Example 3). The aromas of these flavor or fragrance compositions were evaluated by seven expert panelists. In the table, the unit of the blending amount is part(s) by weight.

**[Table 4]**

| Components | Example 4 | Comparative Example 3 |
|---|---|---|
| Ethyl 4-methyl-4-hexenoate | 20.000 | - |
| Dimethyl sulfide | 0.020 | 0.020 |
| Diisopropyl disulfide | 0.050 | 0.050 |
| Ethyl acetate | 30.000 | 30.000 |
| Ethyl butyrate | 5.000 | 5.000 |
| Ethyl hexanoate | 1.000 | 1.000 |
| Ethyl octanoate | 0.600 | 0.600 |
| cis-3-hexenyl acetate | 1.500 | 1.500 |
| cis-3-hexenol | 15.000 | 15.000 |
| trans-3-cis-6-nonadienal | 0.150 | 0.150 |
| 2-phenylethyl alcohol | 0.010 | 0.010 |
| γ-decalactone | 0.200 | 0.200 |
| Ethyl maltol | 2.000 | 2.000 |
| Isobutyric acid | 2.500 | 2.500 |
| Hexanoic acid | 0.800 | 0.800 |
| Propylene glycol (solvent) | 921.170 | 941.170 |
| Total | 1000.000 | 1000.000 |

As a result of the evaluation, all the seven expert panelists reported that the flavor or fragrance composition in Example 4 exhibited a mango-like fragrance note with a higher palatability and a higher natural feeling than the flavor or fragrance composition in Comparative Example 3.

### [Example 5]

### (Shampoo)

Using the fruity strawberry-like fragrance composition obtained in Example 2, a shampoo was prepared according to the formulation shown in Table 5 below. In the table, the unit of the blending amount is part(s) by weight. As a result, it was possible to obtain a shampoo having a natural feeling and exhibiting a fruity strawberry-like fragrance note with excellent palatability.

**[Table 5]**

| Components | Blending amount |
|---|---|
| Triethanolamine laureth sulfate | 16.00 |
| Lauryl dimethylamine oxide | 5.00 |
| Lauryl dimethylaminoacetic acid betaine | 5.00 |
| Ethylene glycol distearate | 3.00 |
| Propylene glycol | 3.00 |
| Sodium benzoate | 0.50 |
| Fruity strawberry-like fragrance flavor or fragrance composition (Example 2) | 0.50 |
| Purified water | the balance |
| Total | 100.00 |

### [Example 6]

### (Body Shampoo)

Using the fruity strawberry-like fragrance composition obtained in Example 2, a body shampoo was prepared according to the formulation shown in Table 6 below. In the table, the unit of the blending amount is part(s) by weight. As a result, it was possible to obtain a body shampoo having a natural feeling and exhibiting a fruity strawberry-like fragrance note with excellent palatability.

**[Table 6]**

| Components | Blending amount |
|---|---|
| Potassium laurate | 10.00 |
| Potassium coconut oil fatty acid | 10.00 |
| Sodium polyoxyethylene dilauryl ether sulfate | 7.00 |
| Ethylene glycol distearate | 3.00 |
| Lauryl hydroxysultaine | 2.00 |
| Propylene glycol | 7.00 |
| Hydroxypropyl methylcellulose | 0.50 |
| Sodium benzoate | 0.30 |
| Disodium EDTA | 0.30 |
| Fruity strawberry-like fragrance flavor or fragrance composition (Example 2) | 1.00 |
| Purified water | the balance |
| Total | 100.00 |

### [Example 7]

### (Toothpaste)

Using the strawberry-flavored flavor or fragrance composition obtained in Example 3, a toothpaste was prepared according to the formulation shown in Table 7 below. In the table, the unit of the blending amount is part(s) by weight. As a result, it was possible to obtain a strawberry flavored toothpaste having a natural feeling and excellent palatability.

**[Table 7]**

| Components | Blending amount |
|---|---|
| Calcium phosphate | 20.00 |
| Sodium carboxymethylcellulose | 1.50 |
| Sodium saccharin | 0.20 |
| Glycerin | 20.00 |
| Sodium lauryl sulfate | 1.00 |
| Hexylene glycol | 5.00 |
| Triclosan | 0.10 |
| Strawberry-flavored flavor or fragrance composition (Example 3) | 1.00 |
| Purified water | the balance |
| Total | 100.00 |

### [Example 8]

### (Strawberry-flavored Drinks)

Using the strawberry-flavored flavor or fragrance composition obtained in Example 3, strawberry-flavored drinks were prepared according to the formulation shown in Table 8 below. In the table, the unit of the blending amount is part(s) by weight. As a result, it was possible to obtain the strawberry-flavored drinks having a natural feeling and excellent palatability.

**[Table 8]**

| Components | Blending amount |
|---|---|
| Glucose-fructose syrup (75%) | 45.00 |
| Citric acid | 5.00 |
| L-ascorbic acid | 0.10 |
| Navel orange juice | 5.00 |
| Strawberry-flavored flavor or fragrance composition (Example 3) | 10.00 |
| Purified water | the balance |
| Total | 100.00 |

### [Example 9]

### (Mango-flavored Chewing Gum)

Using the mango-flavored flavor or fragrance composition obtained in Example 4, a mango-flavored chewing gum was prepared according to the formulation shown in Table 9 below. In the table, the unit of the blending amount is gram (g). As a result, it was possible to obtain a mango-flavored chewing gum having a natural feeling and excellent palatability.

**[Table 9]**

| Components | Blending amount |
|---|---|
| Gum base | 100.00 |
| Sugar | 250.00 |
| Glucose | 40.00 |
| Corn syrup | 60.00 |
| Glycerin | 3.00 |
| Mango-flavored flavor or fragrance composition (Example 4) | 2.00 |

Although the present invention has been described in detail with reference to specific embodiments, it is apparent to those skilled in the art that various changes and modifications can be made without departing from the spirit and scope of the present invention. The present application is based on a U.S. patent application (US63/606, 884) filed on December 6, 2023, which is incorporated by reference in its entirety.

## Claims

1. A flavor or fragrance composition comprising:
at least one of alkyl-substituted 4-pentenoic acid ester derivatives represented by the general formula (1): 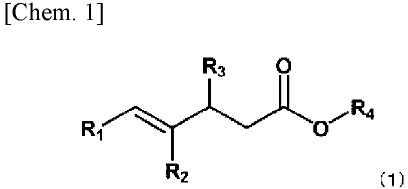
wherein R₁ to R₃ each independently represent a hydrogen atom or an alkyl group having 1 to 4 carbon atoms, and R₄ represents an alkyl group having 1 to 5 carbon atoms or an alkenyl group having 3 to 5 carbon atoms.

2. The flavor or fragrance composition according to claim 1, wherein the alkyl-substituted 4-pentenoic acid ester derivative is ethyl 4-methyl-4-hexenoate represented by the following formula (2).

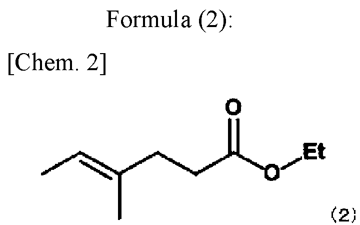

3. The flavor or fragrance composition according to claim 1 or 2, wherein a blending amount of the alkyl-substituted 4-pentenoic acid ester derivative is 0.001 wt% to 30 wt% based on the entire flavor or fragrance composition.

4. A fragrance or cosmetic, a toiletry product, a bath agent, a tobacco product, a food or drink, or a pharmaceutical comprising 0.0001 wt% to 30 wt% of the flavor or fragrance composition according to claim 1 or 2.
